Europäisches Patentamt

European Patent Office

Office européen des brevets

(11) Publication number: 0 262 802
A2

# (12) EUROPEAN PATENT APPLICATION

(21) Application number: 87307671.5

(51) Int. Cl.4: **A61K 37/02**

(22) Date of filing: 28.08.87

The applicant has filed a statement in accordance with Rule 28 (4) EPC (issue of a sample only to an expert). Accession number(s) of the deposit(s): IFO - 14437; FRI - FERM BP - 852.

The microorganism(s) has (have) been deposited with the Institute for Fermentation, Osaka and the Fermentation Research Institute under number(s) 14437 and BP - 852.

(30) Priority: 01.09.86 JP 206577/86
06.08.87 JP 196749/87

(43) Date of publication of application:
06.04.88 Bulletin 88/14

(84) Designated Contracting States:
AT BE CH DE FR GB IT LI LU NL SE

(71) Applicant: **Takeda Chemical Industries, Ltd.**
**27, Doshomachi 2-chome Higashi-ku**
**Osaka-shi Osaka, 541(JP)**

(72) Inventor: **Shiho, Osamu**
**c-502, 3 Sakasedai 1-chome Takarazuka**
**Hyogo 665(JP)**
Inventor: **Sunada, Yukata**
**10-302, 5 Shiroyamacho 1-chome Toyonaka**
**Osaka 561(JP)**
Inventor: **Homma, Mitsuo**
**34-4, Yayoi-cho Itabashi-ku**
**Tokyo 173(JP)**

(74) Representative: **Lewin, John Harvey et al**
**Elkington and Fife High Holborn House 52/54**
**High Holborn**
**London WC1V 6SH(GB)**

(54) Medicament for the treatment or improvement of rheumatoid arthritis or systemic lupus erythematosus.

(57) The pharmaceutical composition of the present invention which comprises an interleukin-2 active substance can produce so marked effects on rheumatoid arthritis or systemic lupus erythematosus that it is of great value for the treatment or improvement of these disease.

EP 0 262 802 A2

## Medicament for the Treatment or Improvement of Rheumatoid Arthritis or Systemic Lupus Erythematosus

BACKGROUND OF THE INVENTION

FIELD OF THE INVENTION

This invention relates to a pharmaceutical composition containing an interleukin-2 (hereinafter referred to as IL-2) active substance which is useful for the treatment or improvement of rheumatoid arthritis or systemic lupus erythematosus.

DESCRIPTION OF THE PRIOR ART

In recent years, as the number of patients with rheumatoid arthritis or systemic lupus erythematosus increases steadily, these disease are becoming more and more important subject of study. These diseases are attracting much attention, because; (1) there are many patients afflicted by the diseases, (2) there is no available treatment based on the cause of the diseases and they are refractory to any treatment. (3) these are chronic systemic diseases calling for long-term treatment, and (4) they are of obscure etiology and offer many phenomena of immunological interest.

Although the true causes of these diseases are yet unknown, it is speculated that some infection or the like may trgger humoral and cellular immunological disorders which in turn cause inflammations not only in the joints but also in various other organs.

The therapeutic drugs available today for rheumatoid arthritis or systemic lupus erythematosus may be classified into a group of symptomatic antiinflammatory agents adapted to suppress inflammatory responses and a group of agents adapted to act on immunological disorders underlying inflammations. However, steroids in common use as anti-inflammatory agents have many side effects such as decreased resistance to infection, moonface, etc., while non-steroidal acidic anti-inflammatory agents also cause many side effects such as gastrointestinal disorder, edema, and so on. Aside from them, gold salts and D-Penicillamin have been used in the treatment of rheumatoid arthritis but these agents are also known to cause many adverse reactions. Therefore, recent years have seen a mounting interest in treatment with agents adapted to alleviate immunological disorders. Levamizole was developed as such an agent. This drug was claimed to improve clinical symptoms by augmenting cellular immunity and suppressor T-cell function, but as serious side effects such as gastrointestinal disorders, neuropathies (insanity, etc.), and blood disorders (granulocytopenia, etc.) were reported, it is seldom used nowadays.

SUMMARY OF THE INVENTION

As aforementioned, there is no adequate therapeutic agent for rheumatoid arthritis or systemic lupus erythematosus.

In researching treatments for these diseases, the present inventors used a human IL-2 active substance having various immunomodulating actions in the treatment of rheumatoid arthritis and systemic lupus erythematosus and found it markedly alleviated or cured these diseases. The finding was followed by further investigations, which have culminated in the present invention.

The present invention is directed to (1) a pharmaceutical composition for the treatment or improvement of rheumatoid arthritis or systemic lupus erythematosus which comprises effective amount of an IL-2 active substance, and a pharmaceutically acceptable carrier, vehicle or diluent therefor, (2) a method for the treatment or improvement of a human patient with rheumatoid arthritis or systemic lupus erythematosus, which comprises administering, as the active ingredient, an effective amount of IL-2 active substance to such patient and (3) use of an IL-2 active substance for the production of a medicament for the treatment or improvement of rheumatoid arthritis or systemic lupus erythematosus.

The IL-2 active substance mentioned above may be any substance having IL-2 activity, that is any substance that is capable of serial passage of T-cells.

Thus, for example, natural IL-2 substances, produced in animal bodies or animal cells, recombinant IL-2 substances which are produced by genetic engineering technique, and any substances related thereto can be employed. Among them, human IL-2 are preferable and human recombinant IL-2 are more preferable. These IL-2 and related substances, when they are proteins, may or may not contain sugar chains.

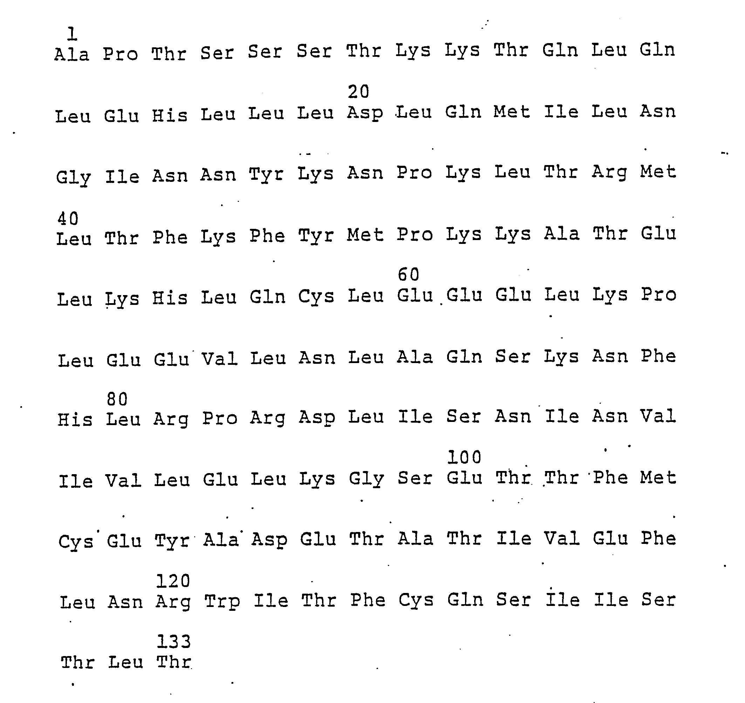

To be specific, a polypeptide (I) having the amino acid sequence of Fig. 1 which is produced by genetic engineering technique [Japanese Unexamined Patent Publication No. 78799/1986 which corresponds to EP Publication No. 176299] or a fragment thereof which has a partial amino acid sequence necessary for its biological or immunological activity. As an example of said fragment, there may be mentioned one which is available on elimination of one amino acid residue (EP Publication No. 91539) or 4 amino acid residues [Japanese Unexamined Patent Publication No. 126088/1985] from the N-terminus of polypeptide (I) or one available on elimination of a few amino acid residues from the C-terminus. Likewise useful is one corresponding to the above-mentioned polypeptide (I) whose amino acid sequence is partly missing or has been partly replaced with one or more other amino acids, for example, one with the cysteine residue in 125-position replaced with a serine residue [Japanese Unexamined Patent Publication No. 93093/1984 which corresponds to U.S. Patent No. 4,518,584].

The aforementioned recombinant IL-2 produced by genetic engineering technique may additionally have a methione residue at the N-terminus of polypeptide (I) [Japanese Unexamined Patent Publication No. 78799/1986 which corresponds to EP Publication No. 176299] or may be a mixture of polypeptide (I) with polypeptide (I) additionally having a methionine residue at the N-terminus [Japanese Unexamined Patent Publication No. 115528/1985 which corresponds to EP Publication No. 145390].

Furthermore, the aforementioned IL-2 may have been chemically modified with polyethylene glycol derivatives, for instance [e.g. Japanese Unexamined Patent Publication No.226821/1985 which corresponds to EP Publication No. 154316 ].

The specific activity of such IL-2 active substances is preferably about 10,000 to 50,000 units/mg and more desirably about 30,000 to 40,000 units/mg. The assay of IL-2 activity and the definition of IL-2 activity unit are as set forth in Japanese Unexamined Patent Publication No. 115528/1985 which corresponds to EP Publication No. 145390.

The IL-2 active substances employed in the present invention are low in toxicity. For example, the IL-2 produced by genetic engineering technique, purified by the procedure described in Japanese Unexamined Patent Publication No. 115528/1985 which corresponds to EP Publication No. 145390 and isolated by the procedure described in Japanese Unexamined Patent Publication No. 78799/1986 which corresponds to EP Publication No. 176299, whose amino acid sequence is as shown in Fig. 1 and whose specific activity is about $3.5 \times 10^4$ units/mg, shows no fatal toxicity when it is administered to cynomolgus monkeys intravenously in a single dose of 6 mg/kg.

Since IL-2 active substances are, thus, low in toxicity, they can be safely administered.

The composition according to the present invention is preferably administered in the form of an injection.

The composition according to the present invention may be made available as an aqueous solution or a solid preparation such as a frozen or lyophylized preparation.

If desired, the composition of the present invention containing an IL-2 active substance may be made available as a mixture with a pharmaceutically acceptable diluent, vehicle, carrier or the like in per se conventional method of pharmaceutical production.

To prepare an aqueous solution for an injection, the composition of the present invention is formulated with, for example, a solvent such as an aqueous medium (e.g. distilled water), an aqueous vehicle (e.g. physiological saline, Ringer's solution), an oleaginous vehicle (e.g. sesame oil, olive oil), etc., if necessary together with solubilizers (e.g. sodium salicylate, sodium acetate), buffers (e.g. sodium citrate, glycerin), isotonicities (e.g. glucose, invert sugar, etc.), stabilizers (e.g. human serum albumin, polyethylene glycol), preservatives (e.g. benzyl alcohol, phenol), soothing agent (e.g. benzalkonium chloride, procaine hydrochloride) and other additives in per se conventional method of pharmaceutical production.

The concentration of said IL-2 active substance or substances in the above aqueous solution is about 1 to 10,000 μg/ml and preferably abut 5 to 50 μg/ml. The concentration in activity units (U) is about 35 to 350,000 U/ml and preferably about 175 to 1,750 U/ml.

This solution is preferably adjusted to pH about 3 to 7 and still more desirably adjusted to pH about 3.5 to 4.5. This adjustment is made, for example, by addition of dilute hydrochloric acid or dilute alkali (e.g. a dilute aqueous solution of sodium hydroxide or sodium hydrogen carbonate).

The composition according to the present invention can be formulated into a solid preparation for an injection which is extemporaneously dissolved for parenteral administration. Such preparations can be manufactured by mixing the IL-2 active substance or substances with, for example, a diluent (e.g. distilled water, physiological saline, aqueous glucose solution), excipients (e.g. carboxymethylcellulose (CMC), sodium alginate), preservatives (e.g. benzyl alcohol, benzalkonium chloride, phenol), soothing agents (e.g. glucose, calcium gluconate, procaine hydrochloride, etc.) and so on in per se conventional method of pharmaceutical production.

It is advantageous to add human serum albumin (hereinafter sometimes referred to briefly as HSA) to the composition of the present invention and adjust the solution to pH about 3 to 7, for the losses of IL-2 activity during storage and in the freezing and lyophylizing procedures are minimized and, in the case of a lyophylized preparation, the reconstituted solution is clear.

HSA may be of any type. For example, it may be a HSA purified form healthy human plasma by Cohn's ethanol fractionation method (fraction No. 6).

The concentration of HSA in the aqueous solution of IL-2 active substance is preferably about 0.1 to 50 mg/ml and still more desirably about 0.5 to 20 mg/ml.

The present composition having immunomodulating actions does in many cases display beneficial effects when used in combination with other drugs and the use of the composition of the present invention in such applications is also subsumed in the concept of the present invention. By way of illustration, addition of the composition of the present invention to, for example, steroids (e.g. dexamethasone isolone) sometimes results in more increased efficacy.

The composition according to the present invention is preferably provided in the form of an aqueous solution, frozen preparation or lyophylized preparation. Particularly, a lyophylized preparation is appropriate.

For the purpose of preventing the attenuation of IL-2 activity, the composition according to the present invention can be manufactured by the following procedures, for instance.

To an aqueous solution of IL-2 active substance, HSA is optionally added in the above-defined concentration range and the mixture is adjusted to an appropriate pH as mentioned hereinbefore. If desired, an isotonicity, a surfactant (e.g. Tween 20, HCO-60) and so on may be further added as mentioned above. When such subsrtances other than HSA are added, the pH of the final solution is adjusted to the aforesaid range by the same procedure as described before. The resulting aqueous solution of the composition according to the present invention can be used as a starting material for the frozen or lyophylized preparation described below.

The frozen composition according to the present invention can be generally manufactured by freezing the above aqueous solution at about -80 to -20°C, for instance. The frozen preparation is preferably stored at about -80 to -20°C.

The lyophylized composition according to the present invention can be manufactured by drying the above frozen preparation under reduced pressure in the conventional manner or freezing the aforementioned aqueous solution or a reconstituted solution from said frozen preparation in small portions in the same manner as above and, then, subjecting the frozen solution to drying under reduced pressure in the conventional manner.

The frozen or lyophylized preparation manufactured in the above manner can be reconstituted with a vehicle, optionally pre-adjusted to an appropriate pH with, for example, hydrochloric acid, to give an aqueous solution of the composition of the present invention.

To prepare the lyophylized composition of the present invention for parenteral use, it is preferable to blend an aqueous solution of IL-2 active substance with an aqueous solution of additives each after passage through a bacterial filter, for instance, for purification, distribute the mixture aseptically into dosage unit containers such as vials, and subject the filled dosage units to the aforementioned lyophilizing procedure. In this procedure, the stability of the composition of the present invention can be enhanced by evacuating the vial or purging with nitrogen gas.

For administration in the form of an injection, the composition of the present invention, when it is an aqueous solution, is used as it is.

When the composition is a lyophilized solid preparation, it is reconstituted with distilled water, physiological saline or the like to give a solution for an injection.

The composition of the present invention can be administered to humans for the treatment or improvement of rheumatoid arthritis or systemic lupus erythematosus.

The composition of the present invention can be administered intravenously, intramuscularly, subcutaneously or intraarticularly and it is preferable to administer intravenously. when it is used for the treatment or improvement of rheumatoid arthritis, it is also preferable to administer intraarticularly.

The dosage of the IL-2 active substance is dependent on the administration route and the severity of the patient's condition. Generally, the dosage can range from about 35 U to about 2,000 U/day/adult patient. When administered intravenously, the preferable range is from about 100 U to about 1,000 U/day/adult patient and more preferable range is from about 100 U to about 500 U/day/adult patient. When administered intraarticularly for the treatment or improvement of rheumatoid arthritis, the preferable range is from about 35 U to about 250 U/day/adult patient and more preferable range is from about 50 U to about 150 U/day/adult patient. However, since the dosage varies with the method and duration of administration, it should not necessarily be limited to the above ranges.

As to the duration of administration, it is preferable to administer the drug for about 5 days to about 3 months. It is generally administered daily for the above duration, however, when administered intraarticularly for the treatment or improvement of rheumatoid arthritis, it is preferable to administer the drug once a week or once every two weeks.

The composition of the present invention can be applicable to patients with rheumatoid arthritis or systemic lupus erythematosus. In particular, it has marked effects on advanced cases which are relatively resistant to steroids and nonsteroidal acidic anti-inflammatory agents.

The use of the composition of the present invention can lead to marked improvements in various symptoms of rheumatoid arthritis or systemic lupus erythematosus in a comparatively short time with a minimum of side effects. Therefore, it represents a great contribution to the fields of internal medicine and orthopedics where these diseases are encountered.

BRIEF DESCRIPTION OF THE DRAWING

Fig. 1 shows the amino acid sequence of the recombinant IL-2 used in the following clinical cases and working examples.

EXAMPLES OF THE PREFERRED EMBODIMENT

The following clinical cases and working examples are further illustrative of the invention and should by no means be construed as limitative thereof, of course.

The IL-2 active substance used in the following clinical cases and working examples is a human IL-2 having alanine at the N-terminus which is produced by cultivating a transformant Escherichia coli N4830/pTB285 (IFO-14437, FERM BP-852)-[Japanese Unexamined Patent Publication No. 78799/1986 which corresponds to EP Publication No. 176299], purified to a high degree of purity by the method described in Japanese Unexamined Patent Publication No. 115528/1985 which corresponds to EP Publication No. 145390 and, then, isolated by the mutual separation procedure described in Japanese Unexamined Patent Publication No. 78799/1986 which corresponds to EP Publication No.176299. Its amino acid sequence is as shown in Fig. 1 and its specific activity is about 3.5 × $10^4$ U/mg. In this specification, one unit represents human IL-2 activity in 1 ml of an arbitrarily selected standard preparation and corresponds to about 28.6 ng of pure human recombinant IL-2.

The above-mentioned transformant has been deposited since April 25, 1985 at the Institute for Fermentation, Osaka, Japan under the accession number of IFO-14437. It has been also deposited since April 30, 1985 at Fermentation Research Institute, Agency of Industrial Science and Technology, Ministry of International Trade and Industry, Japan (FRI) under the accession number of FERM P-8199, and the deposit has been converted to a deposit under the Budapest Treaty, and the above-mentioned microorganism has been stored at FRI under the accession number of FERM BP-852.

Clinical case 1

A 73-year-old man with a 23-year history of definite rheumatoid arthritis (according to The American Rheumatism Association's diagnostic criteria); stage IV (terminal stage: destruction of bone and cartilage, deformation of joints, atrophy of muscle, bony ankylosis), class III (owing to functional disorder, the patient is able to perform only a limited part of work in an ordinary occupation or unable to fully take care of himself), RA test (+) positive.

Chief complaints were pain of joints of the four limbs (shoulders, elbows and knees) and swelling of the left knee joint but the decrease of grip strength (right, 74 mmHg; left, 56 mmHg) was comparatively slight with no morning stiffness and the erythrocyte sedimentation rate, CRP and other laboratory findings were normal, suggesting that the disease was not so active in this case. Previous therapies including the intraarticular injection of steroids as well as the administration of indomethacin and other nonsteroidal anti-inflammatory agents failed to be effective. Therefore, 250 U of IL-2 was intravenously administered for 28 days. This treatment resulted in the disappearance of pain of the shoulder and elbow joints and swelling of the left knee joint.

Clinical case 2

A 27-year-old man with a one and half-year history of definite rheumatoid arthritis; stage II, class II, RA test (+ +) positive. The patient had received nonsteroidal anti-inflammatory agent (diclofenac) therapy for about four months, but still complained of pain of the bilateral knee and shoulder joints and swelling of the hand joints, with morning stiffness (30 min). Therefore, 250 U of IL-2 was intravenously administered first once a day for 48 days and secondly once a week for 5 weeks. As a result, disappearance of morning stiffness, decrease of erythrocyte sedimentation rate (from 35 mm to 15 mm) and decrease of the number of joints with pain (from 9 joints to 2 joints) as well as swelling (from 8 joints to 5 joint) were shown. Thus, clinical symptoms were markedly alleviated.

## Clinical case 3

A 71-year-old woman with a 14-year history of typical rheumatoid arthritis ; stage IV, class III, RA test (+ +) positive. Subjective and objective findings included multiple joint pain and swelling of the bilateral knee and hand joints, with grip strength decreased to 20 mmHg. Fever of 37.7°C and morning stiffness, which was brief in duration (20 min), were noted. Laboratory findings showed marked rheumatic acitivity with a 1-hour erythrocyte sedimentation rate of 130 mm, anemic tendency, hypergammaglobulinemia (2.20 g/dl) and elevation of CRP (14.0). The previous therapy with aspirin and other nonsteroidal anti-inflammatory agents failed to be effective and, therefore, 250 U of IL-2 was intravenously administered for 29 days. As a result, on day 15 after initiation of IL-2 therapy, the patient showed improvements in subjective and objective findings such as alleviation of joint pain, increase of grip strength (right, 80 mmHg; left, 60 mmHg), decrease of duration of morning stiffness (14 min), fall of body temperature (36.9°C), etc. as well as improvement in anemic tendency. By day 29, joint pain had subsided markedly, with the pain disappearing at the elbow and some other joints.

## Clinical case 4

A 77-year-old man with a 10 year history of typical rheumatoid arthritis; stage IV, class III, RA test (+ +) positive. The patient complained of multiple joint pain and swelling of the joints of the hand, with marked morning stiffness (150 min). Decrease of grip strength (right, 40 mmHg; left, 22 mmHg) was also found but the erythrocyte sedimentation rate, body temperature, gammaglobulin, and CRP were normal. Prior to IL-2 therapy, the patient had received oral steroid (prednisolone) therapy for about 2 years without sufficient effects. Therefore, 250 U of IL-2 was intravenously administered for 29 days. As a result, alleviation of pain of the joints of the four limbs was shown by day 29 after initiation of therapy. There was no influence on subjective and objective symptoms or on laboratory findings.

## Clinical case 5

A 71-year-old woman with a 20-year history of typical rheumatoid arthritis; stage IV, RA test (+ +) positive. The patient complained of hard multiple joint pain and his inflammatory symptoms of joints of the four limbs were prominent, with a long duration (420 min) of morning stiffness and markedly decrease of grip strength (right, 26 mmHg; left,28 mmHg). Laboratory examinations also revealed abnormalities in such parameters as 1-hour erythrocyte sedimentation rate (146 mm), $\gamma$-globulin (2.74 g/dl) and CRP (9.7), suggesting its high disease activity. About one and a half year before IL-2 therapy, the patient had been orally treated with nonsteroidal anti-inflammatory agents such as aspirin and a steroid drug (prednisolone) without sufficient effects. Therefore, 500 U of IL-2 was intravenously administered for 28 days. As a result, the joint pain and inflammatory symptoms of joints subsided and a slight decrease of erythrocyte sedimentation rate (115 mm) was shown.

## Clinical case 6

A 69-year-old woman with a 9-year history of typical rheumatoid arthritis; stage IV, caiss III, RA test (+ +) positive. The patient complained of pain of joints of the four limbs but the decrease of grip strength (right, 64 mmHg; left, 72 mmHg) was comparatively slight with no morning stiffness. Previous therapies including administration of indomethacin, naproxen and other nonsteroidal anti-inflammatory agents was relatively effective. When 250 U of IL-2 was intravenously administered for 28 days, this treatment resulted in the decrease of the number of joints with pain (from 16 joints to 5 joints) during the administration as well as the point of pain (from 2.5 point to 1.3 point) by week 1 after completion of IL-2 therapy. Considerable alleviation of pain of joints was shown by IL-2 therapy.

## Clinical case 7

A 33-year-old woman with a 6-year history of typical rheumatoid arthritis; stage III, class II, RA test (+ +) positive. The patient and received chrysotherapy (shiozole) and nonsteroidal anti-inflammatory agent (diclofenac) therapy, but still complained of multiple joint pain and swelling of the bilateral knee and the hand joints. Therefore, 125 U of IL-2 was intravenously administered for 30 days. As a result, pain of the pediphalanx and bilateral shoulder joints disappeared.

### Clinical case 8

A 38-year-old woman with a 5-year history of typical rheumatoid arthritis; stage III, class II, RA test (+) positive. The patient complained of pain and swelling of the bilateral knee. About one and a half year before IL-2 therapy, the patient had been treated with 5 mg/day a prednisolone, 100 mg/day of D-penicillamin and nonsteroidal anti-inflammatory agents without sufficient effect. Therefore, 100 to 150 U of IL-2 was administered in the joint of the knee three times at an interval of one week. As a result, marked alleviation of pain of the joint of the knee was shown.

### Clinical case 9

A 36-year-old woman with systemic lupus erythematosus. Onset 4 years ago. The patient had pain of the upper limb joints, infarct of the skin (+ +), butterfly rash (+), and palmar erythema (+) and showed abnormal values for immunological parameters such as 1-hour erythrocyte sedimentation rate (78 mm), $\gamma$-globulin (3.75 g/dl), IgG (4792 mg/dl), RA test positive, complement fractions $C_3$ (43 mg/dl) and $C_4$ (10 mg/ml), antinuclear antibody (1280 times), and anti-DNA antibody (108 U/ml). Other laboratory values were also abnormal; decrease of white blood cell count (3100/mm³), elevations of GOT (50 U) and GPT (103 U) and occult blood test (+ +) positive. Accordingly, this case was definitely diagnozed as systemic lupus erythematosus. An initial therapeutic attempt with indomethacin and other nonsteroidal anti-inflammatory drugs failed and, therefore, 500 U of IL-2 was intravenously administered for 14 days. As a result, by the end of the above treatment period, disappearance of upper-limb joint pain as well as infarct of the skin, slight decrease of erythrocyte sedimentation rate (69 mm), and normalization of GOT (19 U), GPT (21 U) and occult blood test were shown. By day 14 after completion of IL-2 therapy, improvements were noted in laboratory values, too; $\gamma$-gloubulin (2.18 g/dl), IgG (3151 mg/dl), anti-DNA antibody (26 U/ml) and WBC (6400/mm³).

### Clinical case 10

A 32-year-old woman with systemic lupus erythematosus who had just presented the symptoms. The patient complained of pain of the left wrist and hand joints and dermatologically showed butterfly rash only. Laboratory examinations revealed abnormalities in 1-hour erythrocyte sedimentation rate (27 mm), IgG (1840 mg/dl), antinuclear antibody (1280 times), and anti-DNA antibody (160 times). On the other hand, RA test, GOT, GPT and occult blood test were normal. As the first treatment, single-agent treatment with diclofenac sodium was administered but there was no effect obtained even after 2 weeks. Therefore, 500 U of IL-2 was intravenously administered for 14 days. As a result, the pain of the hand joints disappeared and improvement was also shown in IgG (1707 mg/dl) and anti-DNA antibody (80 times) as well.

### Example 1

In distilled water for injection were dissolved 23 mg/ml of aminoacetic acid, 0.11 ml/ml of 0.1N-hydrochloric acid, 5 mg/ml of human serum albumin and 650 U/ml of human IL-2. The resulting aqueous solution at pH 3.9 was passed through a bacterial filter, distributed in 1 ml portions into vials, and frozen at -40°C. After drying, the vial was purged with $N_2$ gas, capped and pinched. This lyophylized product on re-dissolution in 1 ml of distilled water for injection gave a clear solution and the potency per vial was 653 U. The potency after 2-month storage at 40°C was 664 U.

### Example 2

In distilled water for injection were dissolved 50 mg/ml of mannitol, 0.05 ml/ml of 0.1N-hydrochloric acid, 5 mg/ml of human serum albumin and 650 U/ml of human IL-2. The resulting aqueous solution at pH 3.4 was filtered through a bacterial filter, distributed in 1 ml portions into vials, and frozen at -40°C. After drying, the vial was purged with $N_2$ gas, capped and pinched. This lyophylized product on re-dissolution in 1 ml of distilled water for injection gave a clear solution and the potency per vial was 635 U. The potency after 2-month storage at 40°C was 620 U.

### Example 3

In distilled water for injection were dissolved 15 mg/ml of sorbitol, 35 mg/ml of mannitol, 0.035 ml/ml of 0.1N-hydrochloric acid, 5 mg/ml of human serum albumin and 650 U/ml of human IL-2. The resulting aqueous solution at pH 3.7 was filtered through a bacterial filter, distributed in 1 ml portions in vials, and frozen at -40°C. After drying, the vial was purged with $N_2$ gas, capped and pinched. This lyophilized product on re-dissolution in 1 ml of distilled water for injection gave a clear solution and the potency per vial was 617 U. The potency after 2-month storage at 40°C was 586 U.

## Claims

1. Use of an interleukin-2 active substance for the production of a medicament for the treatment or improvement of rheumatoid arthritis or systemic lupus erythematosus.

2. Use according to claim 1, wherein an interleukin-2 active substance is a human interleukin-2.

3. Use according to claim 2, wherein the human · interleukin-2 is a human recombinant interleukin-2.

4. Use according to claim 1, wherein a medicament is in the form of an injection.

5. Use according to claim 4, wherein a medicament is in the form of an intravenous or intraarticular injection.

6. Use according to claim 4, wherein a medicament is in the unit dosage form to be administered in the dosage from about 35 U to about 2,000 U/day/adult patient in terms of an interleukin-2 active substance.

7. Use according to claim 4, wherein a medicament is in the unit dosage form to be administered intravenously in the dosage from about 100 U to about 1,000 U/day/adult patient in terms of an interleukin-2 active substance.

8. Use according to claim 4, wherein a medicament is in the unit dosage form to be administered intravenously in the dosage from about 100 U to about 500 U/day/adult patient in terms of an interleukin-2 active substance.

9. Use according to claim 4, wherein a medicament is in the unit dosage form to be administered intraarticularly to a human patient with rheumatoid arthritis in the dosage from abut 35 U to about 250 U/day/adult patient in terms of an interleukin-2 active substance.

10. Use according to claim 4, wherein a medicament is in the unit dosage form to be administered intraarticularly to a human patient with rheumatoid arthritis in the dosage from about 50 U to about 150 U/day/adult patient in terms of an interleukin-2 active substance.

11. A pharmaceutical composition for the treatment or improvement of rheumatoid arthritis or systemic lupus erythematosus, which comprises an effective amount of an interleukin-2 active substance, and a pharmaceutically acceptable carrier, vehicle or diluent therefor.

12. The composition according to claim 11, which is in the form of an intravenous or intraarticular injection.

13. The composition according to claim 12, which is in the unit dosage form to be administered in the dosage from about 35 U to about 2,000 U/day/adult patient in terms of an interleukin-2 active substance.

1
Ala Pro Thr Ser Ser Ser Thr Lys Lys Thr Gln Leu Gln

20
Leu Glu His Leu Leu Leu Asp Leu Gln Met Ile Leu Asn

Gly Ile Asn Asn Tyr Lys Asn Pro Lys Leu Thr Arg Met

40
Leu Thr Phe Lys Phe Tyr Met Pro Lys Lys Ala Thr Glu

60
Leu Lys His Leu Gln Cys Leu Glu Glu Glu Leu Lys Pro

Leu Glu Glu Val Leu Asn Leu Ala Gln Ser Lys Asn Phe

80
His Leu Arg Pro Arg Asp Leu Ile Ser Asn Ile Asn Val

100
Ile Val Leu Glu Leu Lys Gly Ser Glu Thr Thr Phe Met

Cys Glu Tyr Ala Asp Glu Thr Ala Thr Ile Val Glu Phe

120
Leu Asn Arg Trp Ile Thr Phe Cys Gln Ser Ile Ile Ser

133
Thr Leu Thr